# Europäisches Patentamt

# European Patent Office

# Office européen des brevets

(19)

(11) Veröffentlichungsnummer: **0 029 203**
**A1**

(12) ## EUROPÄISCHE PATENTANMELDUNG

(21) Anmeldenummer: 80106958.4

(51) Int. Cl.³: **A 61 B 3/10,** A 61 B 3/14

(22) Anmeldetag: 12.11.80

(30) Priorität: 13.11.79 DE 2945744

(71) Anmelder: **Firma Carl Zeiss, Postfach 1369/1380,**
**D-7082 Oberkochen (DE)**

(43) Veröffentlichungstag der Anmeldung: 27.05.81
**Patentblatt 81/21**

(72) Erfinder: **Lang, Walter H., Dr., Finkenweg 33,**
**D-7923 Königsbronn (DE)**
Erfinder: **Muchel, Franz, Dipl.-Phys., Lortzingstrasse 2,**
**D-7923 Königsbronn (DE)**
Erfinder: **Schulz, Kurt, Mozartweg 20,**
**D-7082 Oberkochen (DE)**
Erfinder: **Sümmerer, Gunther, Aalener Strasse 9,**
**D-7082 Oberkochen (DE)**

(84) Benannte Vertragsstaaten: **CH FR GB LI**

(54) **Ophthalmologisches Gerät zur Untersuchung der vorderen und hinteren Augenabschnitte.**

(57) Ophthalmologisches Gerät zur Augenuntersuchung. Es ist eine bekannte Funduskamera (3) mit einer Photospaltlampenbeleuchtung (1) in einem Gerät kombiniert. Damit lässt sich mit einem einzigen Gerät eine Untersuchung und Dokumentation der vorderen und hinteren Augenabschnitte durchführen. Die Photospaltlampenbeleuchtung (1) und die Funduskamera (3) sind sowohl gemeinsam als auch individuell um eine Achse (5 bzw. 10) schwenkbar.

0029203

Ophthalmologisches Gerät zur Untersuchung der vorderen und hinteren Augenabschnitte

Die Erfindung betrifft ein ophthalmologisches Gerät zur Augenuntersuchung.

In der ophthalmologischen Diagnose werden bisher die Untersuchung der vorderen Augenabschnitte (Hornhaut, Vorderkammer, Linse) und die der hinteren Augenabschnitte (Glaskörper, Netzhaut) voneinander getrennt durchgeführt. Für die Untersuchung und Photographie der vorderen Augenabschnitte wird die Photospaltlampe eingesetzt und für die Untersuchung der hinteren Augenabschnitte die Funduskamera. Schon von der optischen Ausrüstung her sind beide Geräte teuer und aufwendig. Da infolge der getrennt durchgeführten Untersuchungen an den vorderen und hinteren Augenabschnitten beide Geräte sogar häufig in auseinander liegenden Räumen aufgestellt und benutzt werden, kommen zum apparativen Aufwand für beide Geräte je ein Netzanschlußgerät, ein Instrumententisch mit zugehöriger Kopfstütze, eine Kleinbildkamera mit motorischem Filmtransport und eine Vorrichtung für die Markierung der Untersuchungszeit und der Patientennummer dazu. Diese apparatemäßige Aufwand wird von vielen Benutzern als Nachteil empfunden.

Der Erfindung liegt deshalb die Aufgabe zugrunde, den apparatemäßigen Aufwand für die Untersuchung der vorderen und hinteren Augenabschnitte zu verringern.

Diese Aufgabe wird erfindungsgemäß dadurch gelöst, daß eine Photospaltlampenbeleuchtung und eine Funduskamera zu einem Gerät für die Untersuchung und Photographie der vorderen und hinteren Augenabschnitte kombiniert sind. Zweckmäßigerweise ist bei einem derartigen Kombinationsgerät ein stereoskopischer Einblick für die Funduskamera vorgesehen.

Ein vorteilhaftes Ausführungsbeispiel des erfindungsgemäßen Kombinationsgerätes zeichnet sich dadurch aus, daß die Photospaltlampenbeleuchtungsgruppe mechanisch mit der Funduskamera gekoppelt ist und daß die Photospaltlampenbeleuchtungsgruppe und die Funduskamera um eine Drehachse gemeinsam und individuell schwenkbar sind, wobei der Winkel zwischen der optischen Achse der Funduskamera und der optischen Achse der Spaltlampe variabel ist. Bei dieser Art von Gerätekombination kann eine vorhandene Funduskamera mit relativ geringem Aufwand in eine Photospaltlampen-Funduskamera umgerüstet werden.

Wenn die Möglichkeit der Neuausstattung eines Untersuchungsplatzes gegeben ist, kann die Photospaltlampen-Funduskamera als Kompaktgerät konzipiert werden, das sich dadurch auszeichnet, daß die Beleuchtungsgruppe der Funduskamera zur Spaltbeleuchtung und -abbildung dient, wobei die Beleuchtungsgruppe und der Beobachtungsteil der Funduskamera um eine Drehachse gemeinsam und individuell schwenkbar sind.

Die mit der Erfindung erzielten Vorteile bestehen insbesondere darin, daß der apparative Aufwand für die Augenuntersuchung beträchtlich gesenkt werden kann, daß der Platzbedarf des Augenarztes für die Untersuchungsgeräte reduziert wird und daß bei Vorhandensein einer Funduskamera diese mit geringem Aufwand zu einer vollwertigen Photospaltlampen-Funduskamera ausgebaut werden kann.

Ein Ausführungsbeispiel der Erfindung ist in der Zeichnung dargestellt und wird im folgenden näher beschrieben. Es zeigen:

Fig. 1    die kombinatorische Verbindung einer Photospaltlampenbeleuchtungsgruppe mit einer Funduskamera in einer schematisierten Schnittgarstellung;

Fig. 2    die Draufsicht auf das in Fig. 1 dargestellte Gerät;

Fig. 3    eine schematische Schnittdarstellung eines Kombinations-Kompaktgerätes, bei dem die Spaltlampenbeleuchtung in die Beleuchtung der Funduskamera integriert ist.

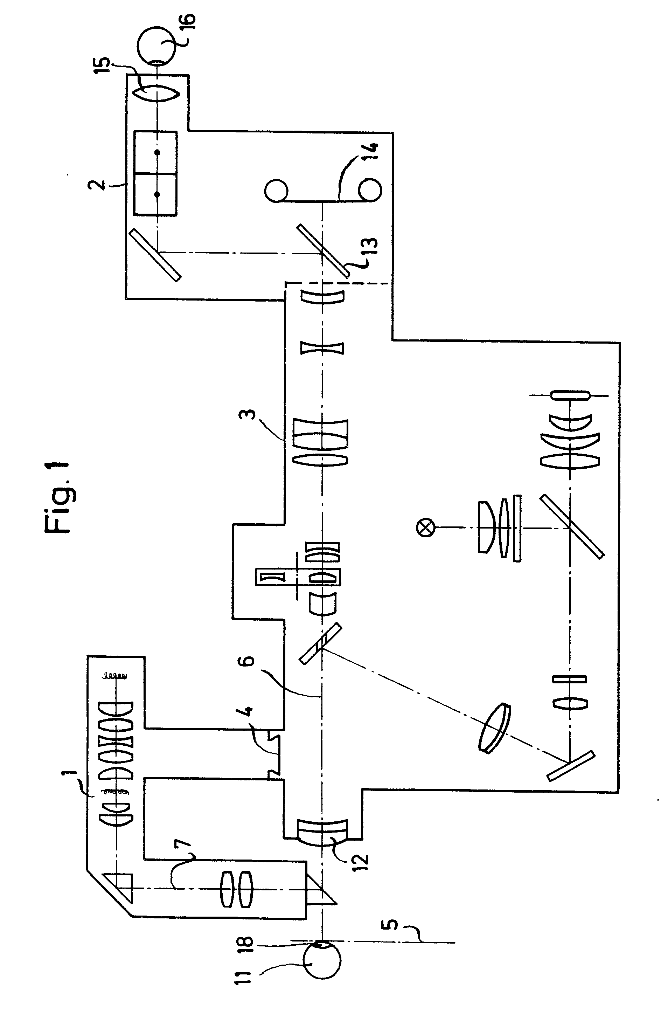

In der Abbildung 1 ist mit 1 die Beleuchtungsgruppe einer Photospaltlampe bezeichnet, wie sie beispielsweise in dem Prospekt 30-241-d/Ma III/74 Noo der Firma Carl Zeiss beschrieben ist. Mit 2 und 3 sind die Baueinheiten

0029203

einer Funduskamera bezeichnet, die beispielsweise aus dem Prospekt 30-245.1-d/MA X/77 Koo der Firma Carl Zeiss oder aus der DOS 27 41 992 bekannt ist, wobei die Beleuchtungsgruppe und die Beobachtungsgruppe als Baueinheit 3 und der Okularkopf als Baueinheit 2 zusammengefaßt sind. Die Photospaltlampenbeleuchtungsgruppe 1 kann über das Verbindungsteil 4 mit der Funduskamera 2,3 mechanisch gekoppelt werden. Mit der Kopplung verbunden ist ein achsiales Verschieben der Funduskamera vom Auge 11 des Patienten weg, damit das Objektiv 12 der Funduskamera auf die vorderen Augenabschnitte fokussiert ist. Im Okularkopf 2 der Funduskamera ist ein Umlenkspiegel 13 vorgesehen, der den Beobachtungsstrahlengang entweder auf eine Dokumentationsvorrichtung 14 oder über eine binokulare Beobachtungsvorrichtung 15 zum Beobachter 16 lenkt. Die Photospaltlampenbeleuchtungsgruppe 1 und die Funduskamera 2,3 sind um die Drehachse 5 gemeinsam oder individuell schwenkbar.

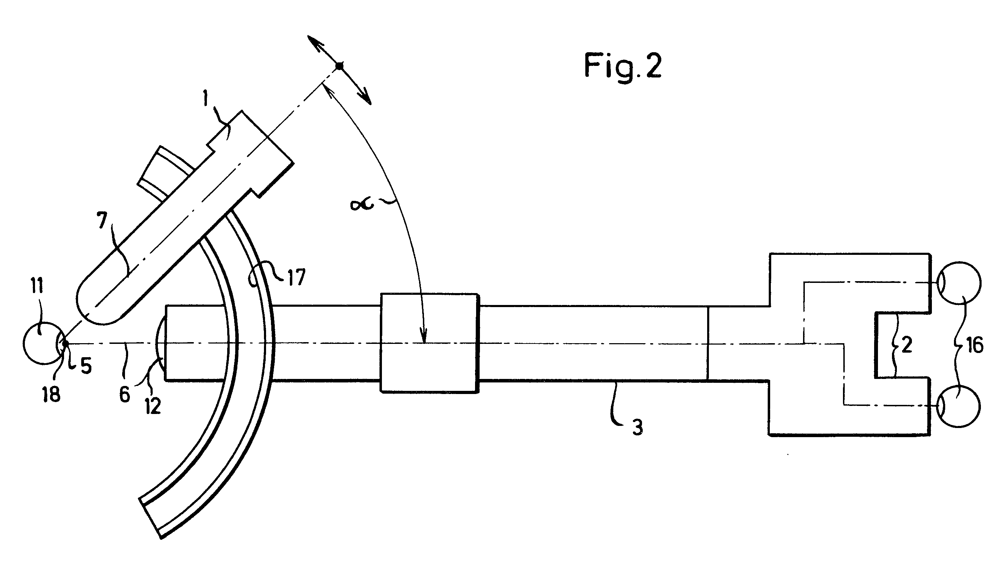

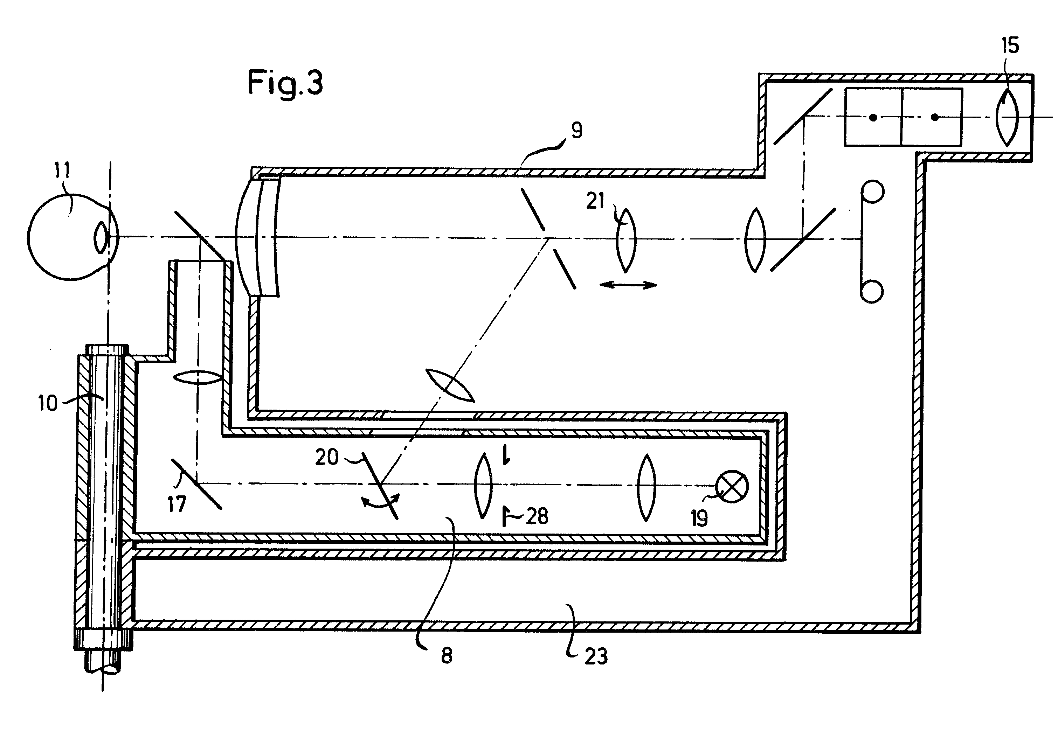

In der in Fig. 2 dargestellten Draufsicht ist der Bogen 17 zu sehen, auf dem die Spaltlampenbeleuchtungsgruppe 1 zur Untersuchung der vorderen Augenabschnitte 18 geführt wird. Der Einstellwinkel $\alpha$ ist damit beliebig einstellbar. In dem in Fig. 3 dargestellten Ausführungsbeispiel ist der Beobachtungsteil der Funduskamera und ein Teil der Beleuchtungsgruppe in der Baueinheit 9 untergebracht. Die Baueinheit 9 ist über ein Verbindungsteil 23 mit der Achse 10 verbunden. In der Baueinheit 8 befindet sich der Teil der Beleuchtungsgruppe der Funduskamera, der auch für die Spaltbeleuchtung verwendet wird. Im Falle der Verwendung als Photospaltlampe wird in dem Blendenraum 28 der Spalt eingebracht, der von der Lampe 19 beleuchtet wird und über die Beleuchtungsoptik der Gruppe 8 auf die vorderen Augenabschnitte abgebildet wird. In diesem Fall wird der Spiegel 20 aus dem Strahlengang herausgeklappt oder an die Stelle des Spiegels 17 gesetzt.

Bei Verwendung des Kombinationsgerätes als Funduskamera bleibt der Spiegel 20 an seiner Stelle. Die Linse 21 kann achsial verschoben werden, um die Beobachtungsoptik auf die hinteren Augenabschnitte zu fokussieren. Die optischen Baugruppen 13 und 8 sind gemeinsam und individuell um die Achse 10 schwenkbar.

0029203

Das in den Figuren 1 und 2 dargestellte Ausführungsbeispiel für die Kombination einer vorhandenen Funduskamera mit einer Spaltlampenbeleuchtungsgruppe ist nicht auf die gezeigte stehende Anordnung beschränkt. Eine derartige Kombination ist vielmehr auch mit hängend angeordneten Gerätegruppen möglich. Wesentlich dabei ist, daß eine mechanische Verbindung zwischen der Spaltlampenbeleuchtungsgruppe und der Funduskamera herstellbar ist, und daß beide Kombinationsteile um eine Drehachse gemeinsam und individuell schwenkbar sind.

0029203

Patentansprüche:

1. Ophthalmologisches Gerät, dadurch gekennzeichnet, daß eine Photospalt-lampenbeleuchtung (1) und eine Funduskamera (3) zu einem Gerät für die Untersuchung und Photographie der vorderen und hinteren Augenabschnitte kombiniert sind.

2. Ophthalmologisches Gerät nach Anspruch 1, dadurch gekennzeichnet, daß für die Funduskamera ein stereoskopischer Einblick (2) vorgesehen ist.

3. Ophthalmologisches Gerät nach Anspruch 2, dadurch gekennzeichnet, daß die Photospaltlampenbeleuchtungsgruppe (1) über ein Verbindungsteil (4) mechanisch mit der Funduskamera gekoppelt ist und daß Photospaltlampenbeleuchtungsgruppe und Funduskamera um eine Drehachse (5) gemeinsam und individuell schwenkbar sind, wobei der Winkel (α) zwischen der optischen Achse (6) der Funduskamera und der optischen Achse (7) der Spaltlampe variabel ist.

4. Ophthalmologisches Gerät nach Anspruch 1, dadurch gekennzeichnet, daß für ein Kompaktgerät die Beleuchtungsgruppe (8) der Funduskamera zur Spaltbeleuchtung und -abbildung dient, wobei die Beleuchtungsgruppe (8) und der Beobachtungsteil (9) der Funduskamera um eine Drehachse (10) gemeinsam und individuell schwenkbar sind.

Fig. 1

Fig.2

Fig.3

**0029203**
Nummer der Anmeldung

EP 80 10 6958

Europäisches
Patentamt

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| X | US - A - 3 944 342 (M. MARTINEZ)<br><br>* Zusammenfassung; Spalte 2, Zeilen 55-64; Spalte 3, Zeilen 30-35; Spalte 3, Zeile 51 - Spalte 4, Zeile 29; Spalte 4, Zeilen 43-53; Spalte 7, Zeilen 9-22; Abbildung 1 *<br><br>-- | 1-4 |
| X | FR - A - 1 238 908 (R.A. DUDRAGNE)<br><br>* Seite 1, linke Spalte , Zeilen 1-5; Seite 1, linke Spalte, Zeile 26 - Seite 1, rechte Spalte, Zeile 4; Seite 2, linke Spalte, Zeilen 12-34; Seite 2, linke Spalte, Zeile 56 - Seite 3, linke Spalte, Zeile 2; Abbildungen 1,2 *<br><br>-- | 1-4 |
| | US - A - 4 068 932 (S. OHTA et al.)<br><br>* Zusammenfassung; Spalte 2, Zeile 49 - Spalte 3, Zeile 4; Spalte 3, Zeile 33 - Spalte 4, Zeile 68; Abbildungen 1,2 *<br><br>& DE - A - 2 707 788<br><br>-- | 1 |
| | US - A - 4 146 310 (Y. KOHAYAKAWA et al.)<br><br>* Zusammenfassung; Spalte 3, Zeile 6 - Spalte 4, Zeile 45; Abbildungen 1,2 *<br><br>---- | 1 |

**KLASSIFIKATION DER ANMELDUNG (Int. Cl.³)**

A 61 B 3/10
3/14

**RECHERCHIERTE SACHGEBIETE (Int. Cl.³)**

A 61 B 3/10
3/12
3/14

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 18-02-1981 | BEAVEN |

EPA form 1503.1   06.78